# EUROPEAN PATENT SPECIFICATION

(11) **EP 1 124 946 B1**
(45) Date of publication and mention of the grant of the patent: **20.06.2007**
(21) Application number: 99947259.0
(22) Date of filing: 12.10.1999
(51) Int. Cl.: C12N 15/00, C12N 9/96, C07K 1/06

(54) **GLYCOSYLATED PROTEINS HAVING REDUCED ALLERGENICITY**
GLYKOSYLIERTE PROTEINE MIT REDUZIERTER ALLERGENITÄT
PROTEINES GLYCOSYLEES A ALLERGENICITE REDUITE

(30) Priority: 30.10.1998 DK 981401; 25.11.1998 DK 981551; 17.05.1999 DK 990682; 04.10.1999 DK 991419
(43) Date of publication of application: 22.08.2001
(73) Proprietor: Novozymes A/S, 2880 Bagsvaerd (DK)
(72) Inventor: OLSEN, Arne, Agerlin, 2880 Bagsvaerd (DK); ROGGEN, Erwin, Ludo, 2880 Bagsvaerd (DK); ERNST, Steffen, 2880 Bagsvaerd (DK)
(74) Representative: Garde, Charlotte Kammer
(86) International application number: PCT/DK1999/000540
(87) International publication number: WO 2000/026354

(56) References cited:
- WO-A1-88/01647
- WO-A1-97/08322
- US-A- 5 041 376
- US-A- 5 585 250
- US-A- 5 766 897
- MICHAEL S. HERSHFIELD.: 'Use of site-directed mutagenesis to enhance the epitope.shielding effect of covalent modification of proteins with polyethylene glycol' PROC. NATL. ACAD. SCI. USA, vol. 88, August 1991, pages 7185 - 7189, XP002129652
- DATABASE DERWENT BIOTECHNOLOGY ABS [Online] REGHEERE G.: 'Glycoproteins of pharmaceutical use: importance of good glycosylation - for maintaining good biological conformation etc., recombinant DNA technology', XP002946111 Retrieved from Dialog Information service, File 357, accession no. 0056432 Database accession no. 87-00780 & BIOFUTUR vol. 48, 1986, pages 55 - 62, XP008040783

## Description

The present invention relates to the field of immunology, in particular allergenicity. More specifically it relates to the reduction of allergenicity by the in vivo N-glycosylation of proteins.

### Background of the invention

Proteins, including enzymes, are produced on a large industrial scale for application in a variety of industrial fields. Today detergent, food as well as pharmaceutical industries are benefiting from the development of modern protein and enzyme technologies. However, a major drawback associated with the increased exposure to proteins is the risk of allergic responses in susceptible individuals.

Prevention of allergy in susceptible individuals is therefore a research area of great importance. The general mechanism behind an allergic response is divided into a sensitisation phase and a symptomatic phase. The sensitisation phase involves a first exposure of an individual to an allergen. This event activates T- and B-lymphocytes, and leads to the production of allergen specific immunoglobulin E (IgE) antibodies. The symptomatic phase is initiated by a second exposure to the same or a resembling antigen. The specific IgE antibodies bind among others to specific IgE receptors on mast cells and basofils, while simultaneously capturing the allergen. The polyclonal nature of this process results in bridging and clustering of the IgE receptors, and subsequently in the activation of mast cells and basophils. In turn this activation triggers the release of various chemical mediators involved in the early as well as late phase reactions of the symptomatic phase. It seems there is a correlation between the degree of IgE binding and the severity of the allergy reaction. In the present context the denotation of antibodies follow the standard denotation, i.e. IgE is immunoglobulin E and so forth.

Before the above mentioned specific immune response comes into play, the body will try to remove the invaders by non-specific mechanisms. In the lungs and liver, for example, and probably also in the skin, resident macrophages function as scavenger cells. Scavengers are phagocytically active cells that are equipped to capture and digest foreign antigens, for example bacteria. To improve their capturing efficiency they express on their surface scavenger receptors as well as high-mannose receptors, whereto anionic and mannose-containing structures normally expressed on the bacterial surface bind.

It is believed that reducing the binding of pre-existing free and cell-bound epitope specific as well as cross-reacting IgE antibodies, and reducing the production of IgE antibodies by enhancing the recognition of antigens by phagocytic cells are factors that will help the body control an allergic reaction to proteins.

One way of achieving this is to modify the protein responsible for the allergic reaction. Such protein modifications have been described in the prior art. In WO 96/17929, WO 96/40792, WO 97/30148 and WO 98/35026 proteins were modified chemically by the addition of polyethylen glycol. The antigens may be chemically altered to reduce the binding by antibodies. The antigen derivatives revealed a reduced binding of specific antibodies, a reduced antigenicity as indicated by the production of IgG1 antibodies, and basically no allergenicity as suggested by the absence of detectable specific IgE antibody levels.

Although effective, the process of chemically modifying proteins, i.e. by PEG-ylation is rather time consuming and thus costly. The present invention reveals a method for optimising the in vivo modification of proteins with glycosyl oligomers with the aim to reduce the allergenicity of proteins. This approach may reduce production costs five fold.

WO 98/1337 describes a method whereby the uptake of soluble peptides by dendritic cells is enhanced by the addition of mannose residues to the peptides. This clearly illustrates that it is possible to manipulate an immune response to a soluble antigen by chemically adding sugars, such as mannose.

US A 5,041,376 discloses a method to identify or shield functional sites or epitopes by introduction of one single additional non-native N-glycosylation site. Among a range of methods to assess structural and enzymatic properties of the glycosylated variants, this patent uses a monoclonal antibody assay to evaluate if the introduction of a glycosylation site has reduced antibody binding.

Babiker et al, J.Agric. Food Chem. 1998, 46, 866-871, describes a soy protein-galactomannan conjugate prepared by the Maillard reaction which removed the allergenicity of the 34kDa protein which is frequently recognized by the IgE antibody in the sera of soybean- sensitive patients as a major allergen.

In the present context the terms allergy, allergenic and allergenicity is used according to their usual definitions, i.e. to describe the reaction due to immune responses wherein the antibody most common is IgE, less often IgG4 and diseases due to these immune responses. Allergenic diseases include urticaria, hay fever, asthma, and atopic dermatitis. Occassionally, these diseases may evolve into anaphylactic shock.

### Summary of the invention

This invention relates to a method according to claim 1.

Further embodiments of the method are set out in claims 2 to 10.

Thus in one embodiment of the present invention the allergenicity of the glycosylated protein variant is less than 50%, preferably less than 25%, more preferably less than 1% of the parent protein's allergenicity as defined in claim 1.

A glycosylated protein variant having at least one additional glycosylation site allowing shielding of at least one epitope of the parent protein, said glycosylated protein variant exhibiting substantially the same functionality as the parent protein may be produced by the method of the invention.

### Drawings

Figure 1 shows the 3D picture of two constructed lipolase variants.
Figure 2 is a gel showing a wild type lipolase and the two lipolase variants depicted in Figure 1.
Figure 3 shows the integrated antibody response in IT-rat model.
Figure 4 shows the integrated antibody response level in sc-mouse model.

### Detailed disclosure of the invention.

The object of the present invention is to provide a novel method of producing proteins having reduced allergenicity by in vivo glycosylation. The object is achieved by the construction of a DNA molecule encoding the protein variant, where the DNA molecule has at least one sub-sequence encoding a additional glycosylation site.

The essence of the present invention is that glycosylation provides a soluble antigen with attachment sites for binding by resident macrophages e.g. alveolar macrophages of the lung. Glycosylation introduces new epitopes but glycosyl-specific antibodies rarely if ever belong to the IgE class. On the contrary, the binding by macrophages redirects the specific immune response to the antigen away from an allergic response.

At the same time glycosylation provides shielding of the antigen from circulating antibodies capable of reacting with the protein part of the antigen. The shielding of IgE epitopes contributes to a reduction in allergenicity. By the term shielding is meant that the glycosyl chain of the glycosylated protein physically is "covering" at least one epitope of the parent protein. This shielding, or covering prevents the specific antibody recognition of the epitope. The efficacy of the binding by macrophages as well as the degree to which the epitopes are shielded from being recognised by antibodies, i.e. if one or more epitopes are shielded depend entirely on the nature of the glycosyl chain, i.e. on the complexity and mannose content of the glycosyl chain, and on whether the glycosyl chain is branched or linear.

The inventor of the present invention has surprisingly found that by using the claimed method, which includes the in vivo glycosylation of proteins, the allergenicity of the proteins is greatly reduced when determined after intratracheal exposure, but that the allergenicity of the proteins is reduced to a much lower extent when determined after subcutaneous exposure, indicating that the mechanism of reduced allergencicity may be dominated by macrophages resident in the lung alveolar. It follows that by using the present method of producing a glycosylated protein it is possible to achieve a higher yield of an end product having reduced allergenicity as compared to the conventional chemical prior art protein modifications. This is due to the fact that the end product is produced in vivo and therefore does not require in vitro modification and purification.

According to the present invention the glycosylated protein variant produced is an N-glycosylated protein variant. N-glycosylation is found at sites of the sequence Asn-Xaa-Ser, Asn-Xaa-Thr, or Asn-Xaa-Cys, in which neither the Xaa residue nor the aminoacid following the tri-peptide consensus sequence is a proline (T. E. Creighton, 'Proteins - Structures and Molecular Properties, 2nd edition, W.H. Freeman and Co., New York, 1993, pp. 91-93) . It is thus desirable to introduce such recognition sites in the sequence of the parent protein. The specific nature of the glycosyl chain of the glycosylated protein variant may be linear or branched depending on the specific requirements and properties of the protein variant.

An important feature of the present invention is that the glycosylated protein variant exhibits substantially the same functionality as the parent protein.

The term "substantially the same functionality" indicates a protein variant as a structural analogue of the parent protein with equivalent biological functions or even better yet having a glycosylation modification when compared to the parent protein. It is possible to assess whether the modifications will interfere substantially with the functionality of the protein variant either by computer modelling of the desired substitions or by measuring the biological activity of the glycosylated protein variant, e.g. the enzymatic activity of a glycosylated protein.

In another aspect of the present invention the allergenicity of the glycosylated protein variant is at least 50% lower than the parent protein allergenicity expressed as the levels of IgE antibodies developed in animals appropriately exposed to the antigen as defined in Claim 1. Preferably thus the allergenicity of the protein variant in test animals is substantially none when compared to the wild-type allergenicity.

In addition to the reduction in allergenicity caused by the recognition by macrophages of the glycosylated protein variants with additional glycosylation group (s), the additional glycosylation may shield at least one surface epitope of the protein leading to lowered antigenicity and hence, the possibility of a further reduction of the allergenicity.

In the present context, a "surface epitope" is to be understood as a small region on a protein surface which is recognised and bound by antibodies specific for that protein.

According to the present invention the reduction in antibody binding to the protein variant may be at least a 30 % reduction in antibody binding, preferably 45 % reduction in antibody binding, with the proviso that the function of the protein variant is not substantially impaired. The reduction in antibody binding may be measured using competitive ELISA.

In order to enhance the epitope shielding effect of the additional glycosylation sites, these can preferentially be introduced in the vicinity of epitopes on the protein surface.

The identification of the epitope(s) may be achieved by established methods such as those disclosed in WO 92/10755 (by U. Løvborg), by Walshet et al, J. Immunol. Methods, vol. 121, 1275-280, 1989, and by Schoofs et al. J. Immunol. vol. 140, 611-616, 1987, Kohlman et al., Protein Science, Vol. 8 (Suppl. 2), page 68, 1999.

In a preferable mode, the identification of the epitope(s) may be achieved by screening of phage display libraries. A phage is a virus that infects bacteria. The principle behind phage display is that a heterologous DNA sequence can be inserted in the gene coding for a coat protein of the phage. The phage will make and display the hybrid protein on its surface where it can interact with specific target agents. Such target agent may be an epitope-specific antibody. It is therefore possible to select specific phages from the bulk of million of phages, each expressing their own hybrid protein. A random peptide display package library is a library displaying peptides of predetermined lengths, for example 9 amino acids long. The peptides of the hybrid proteins of the specific phages which bind protein-specific antibodies define the epitopes of that particular parent protein. The corresponding residues of the parent protein can be found by comparing the selected peptide sequences with the amino acid sequence and 3-dimensional structure of the parent protein. In order to enhance the shielding effect of the additional glycosylation groups these can be introduced in the vicinity of surface epitopes identified in this manner.

### Finding suitable substitutions:

When choosing suitable substitutions to introduce N-glycosylation sites, it is desirable to search for Asn, Ser, Thr, or Cys residues that could be part of an Asn-Xaa-Ser/Thr/Cys consensus sequence. Completing the consensus sequence by site-directed mutagenesis will require only one substitution, and thus be less disruptive to the protein folding and function. Further, computer modeling can be employed to ensure that the amino acid substitutions lead to stable protein variants. Generally, conservative substitutions, and substitiutions which will lead to the side chain of the Asn residue of the glycosylation consensus sequence being accessible to solvent are preferred. The accessibility to the solvent of amino acid side chains can be determined by applying the DSSP computer program to the relevant part of the protein structure. The DSSP program is disclosed in W. Kabsch and C. Sander, BIOPOLYMERS 22 (1983) pp. 2577-2637)

In the case of choosing substitutions, which are intended to shield an identified epitope, one can evaluate by computer modeling using standard techniques, how the N-linked carbohydrate moiety is predicted to shield the epitope in question. Several software packages exist which may be employed to make this evaluation. One example is the Insight suite of modeling software from MSI, Inc.

In a preferred embodiment of the invention, more than one additional glycosylation site is introduced. In that case, it may be difficult to assess a priori how well the functionality of the protein is maintained while antigenicity and/or allergenicity is reduced. This can be achieved by establishing a library of diversified mutants each having one or more additional glycosylation sites introduced and selecting those variants which show good retention of function and at the same time a good reduction in antigenicity. In the case of lipase, this can be tested by assaying the secreted, glycosylated lipase variants for enzyme activity and antigen binding by competitive ELISA, as described below in the experimental section, however, the scope of the invention is by no means limited to lipase, which serves only to provide an example. A diversified library can be established by a range of techniques known to the person skilled in the art (Reetz MT, Jarger KE; Biocatalysis, Discovery to Application edited by Fessner WD, Vol. 200, pp. 31-57, 1999, Stemmer, Nature, vol. 370, p.389-391, 1994, or Zhao and Arnold, Proc. Natl. Acad. Sci.; USA, vol. 94, pp 7997-8000, 1997 or Yano et al., Proc. Natl. Acad. Sci., USA, vol. 95, pp 5511-5515, 1998). In a more preferable embodiment, substitutions are found by a method comprising the following steps: 1) a range of additional glycosylation sites are listed, 2) a library is designed which introduces a randomized subset of these additional glycosylation sites in the target gen, 3) the library is expressed, and preferred variants are selected. In a most preferred embodiment, this method is supplemented with additional rounds of screening and/or family shuffling of hits from the first round of screening (J.E. Ness, et al., Nature Biotechnology, Vol. 17, pp. 893-896, 1999) and/or combination with other methods of reducing allergenicity by genetic means (such as that disclosed in WO 92/10755).

The parent protein may be any protein. The term parent protein includes any protein, such as a wild-type protein or a variant thereof having a functionality identical to the parent protein.

### Pharmaceutical polypeptides

The term "pharmaceutical polypeptides" is defined as polypeptides, including peptides, such as peptide hormones, proteins and/or enzymes, being physiologically active when introduced into the circulatory system of the body of humans and/or animals.
Pharmaceutical polypeptides are potentially immunogenic as they are introduced into the circulatory system.
Examples of "pharmaceutical polypeptides" contemplated according to the invention include insulin, ACTH, glucagon, somatostatin, somatotropin, thymosin, parathyroid hormone, pigmentary hormones, somatomedin, erythropoietin, luteinizing hormone, chorionic gonadotropin, hypothalmic releasing factors, antidiuretic hormones, thyroid stimulating hormone, relaxin, interferon, thrombopoietin (TPO) and prolactin.

Accordingly, the method according to the invention may be applied to proteins used for industrial purposes, such as enzymes.

In a preferred embodiment of the invention the parent protein is an enzyme and may be selected from the group of enzymes consisting of proteases, lipases, phytases, polysaccharide lyases, oxidoreductases, transglutaminases, glycosyl hydrolases, and glycoseisomerases, in particular as mentioned in the following.

### Parent Proteases

Parent proteases (i.e. enzymes classified under the Enzyme Classification number E.C. 3.4 in accordance with the Recommendations (1992) of the International Union of Biochemistry and Molecular Biology (IUBMB)) include proteases within this group.

Examples include proteases selected from those classified under the Enzyme Classification (E.C.) numbers:
3.4.11 (i.e. so-called aminopeptidases), including 3.4.11.5 (Prolyl aminopeptidase), 3.4.11.9 (X-pro aminopeptidase), 3.4.11.10 (Bacterial leucyl aminopeptidase), 3.4.11.12 (Thermophilic aminopeptidase), 3.4.11.15 (Lysyl aminopeptidase), 3.4.11.17 (Tryptophanyl aminopeptidase), 3.4.11.18 (Methionyl aminopeptidase).
3.4.21 (i.e. so-called serine endopeptidases), including 3.4.21.1 (Chymotrypsin), 3.4.21.4 (Trypsin), 3.4.21.19 (Glutamyl Endopeptidase Glu-C, V8 from Staphylococcus aureus), 3.4.21.25 (Cucumisin), 3.4.21.32 (Brachyurin), 3.4.21.48 (Cerevisin) and 3.4.21.62 (Subtilisin);
3.4.22 (i.e. so-called cysteine endopeptidases), including 3.4.22.2 (Papain), 3.4.22.3 (Ficain), 3.4.22.6 (Chymopapain), 3.4.22.7 (Asclepain), 3.4.22.14 (Actinidain), 3.4.22.30 (Caricain) and 3.4.22.31 (Ananain);
3.4.23 (i.e. so-called aspartic endopeptidases), including 3.4.23.1 (Pepsin A), 3.4.23.18 (Aspergillopepsin I), 3.4.23.20 (Penicillopepsin) and 3.4.23.25 (Saccharopepsin); and
3.4.24 (i.e. so-called metalloendopeptidases), including 3.4.24.28 (Bacillolysin).
   Examples of relevant subtilisins comprise subtilisin BPN', subtilisin amylosacchariticus, subtilisin 168, subtilisin mesentericopeptidase, subtilisin Carlsberg, subtilisin DY, subtilisin 309, subtilisin 147, thermitase, aqualysin, Bacillus PB92 protease, proteinase K, Protease TW7, and Protease TW3.

Specific examples of such readily available commercial proteases include Esperase®, Alcalase®, Neutrase®, Dyrazym®, Savinase®, Pyrase®, Pancreatic Trypsin NOVO (PTN), Bio-Feed^{™} Pro, Clear-Lens Pro (all enzymes available from Novo Nordisk A/S).

Examples of other commercial proteases include Maxtase®, Maxacal®, Maxapem® marketed by Gist-Brocades N.V., Opticlean® marketed by Solvay et Cie. and Purafect® marketed by Genencor International.

It is to be understood that also protease variants are contemplated as the parent protease. Examples of such protease variants are disclosed in EP 130.756 (Genentech), EP 214.435 (Henkel), WO 87/04461 (Amgen), WO 87/05050 (Genex), EP 251.446 (Genencor), EP 260.105 (Genencor), Thomas et al., (1985), Nature. 318, p. 375-376, Thomas et al., (1987), J. Mol. Biol., 193, pp. 803-813, Russel et al., (1987), Nature, 328, p. 496-500, WO 88/08028 (Genex), WO 88/08033 (Amgen), WO 89/06279 (Novo Nordisk A/S), WO 91/00345 (Novo Nordisk A/S), EP 525 610 (Solvay) and WO 94/02618 (Gist-Brocades N.V.).

The activity of proteases can be determined as described in "Methods of Enzymatic Analysis", third edition, 1984, Verlag Chemie, Weinheim, vol. 5.

### Parent Lipases

Parent lipases (i.e. enzymes classified under the Enzyme Classification number E.C. 3.1.1 (Carboxylic Ester Hydrolases) in accordance with the Recommendations (1992) of the International Union of Biochemistry and Molecular Biology (IUBMB)) include lipases within this group.

Examples include lipases selected from those classified under the Enzyme Classification (E.C.) numbers:
3.1.1 (i.e. so-called Carboxylic Ester Hydrolases), including (3.1.1.3) Triacylglycerol lipases, (3.1.1.4.) Phosphorlipase A2.

Examples of lipases include lipases derived from the following microorganisms. The indicated patent publications are incorporated herein by reference:
Humicola, e.g. H. brevispora, H. lanuginosa, H. brevis var. thermoidea and H. insolens (US 4,810,414)
Pseudomonas, e.g. Ps. fragi, Ps. stutzeri, Ps. cepacia and Ps. fluorescens (WO 89/04361), or Ps. plantarii or Ps. gladioli (US patent no. 4,950,417 (Solvay enzymes)) or Ps. alcaligenes and Ps. pseudoalcaligenes (EP 218 272) or Ps. mendocina (WO 88/09367; US 5,389,536).
Fusarium, e.g. F. oxysporum (EP 130,064) or F. solani pisi (WO 90/09446). Mucor (also called Rhizomucor), e.g. M. miehei (EP 238 023).
Chromobacterium (especially C. viscosum), Aspergillus (especially A. niger).
Candida, e.g. C. cylindracea (also called C. rugosa) or C. antarctica (WO 88/02775) or C. antarctica lipase A or B (WO 94/01541 and WO 89/02916).
Geotricum, e.g. G. candidum (Schimada et al., (1989), J. Biochem., 106, 383-388).
Penicillium, e.g. P. camembertii (Yamaguchi et al., (1991), Gene 103, 61-67).
Rhizopus, e.g. R. delemar (Hass et al., (1991), Gene 109, 107-113) or R. niveus (Kugimiya et al., (1992) Biosci. Biotech. Biochem 56, 716-719) or R. oryzae.
Bacillus, e.g. B. subtilis (Dartois et al., (1993) Biochemica et Biophysica acta 1131, 53-260) or B. stearothermophilus (JP 64/7744992) or B. pumilus (WO 91/16422).

Specific examples of readily available commercial lipases include Lipolase®, Lipolase^{™} Ultra, Lipozyme®, Palatase®, Novozym® 435, Lecitase® (all available from Novo Nordisk A/S).

Examples of other lipases are Lumafast^{™}, Ps. mendocian lipase from Genencor Int. Inc.; Lipomax^{™}, Ps. pseudoalcaligenes lipase from Gist Brocades/Genencor Int. Inc.; Fusarium solani lipase (cutinase) from Unilever; Bacillus sp. lipase from Solvay enzymes. Other lipases are available from other companies.

It is to be understood that lipase variants too are contemplated as the parent enzyme. Examples of such are described in e.g. WO 93/01285 and WO 95/22615.

The activity of the lipase can be determined as described in "Methods of Enzymatic Analysis", Third Edition, 1984, Verlag Chemie, Weinhein, vol. 4, or as described in AF 95/5 GB (available on request from Novo Nordisk A/S).

### Parent Oxidoreductases

Parent oxidoreductases (i.e. enzymes classified under the Enzyme Classification number E.C. 1 (Oxidoreductases) in accordance with the Recommendations (1992) of the International Union of Biochemistry and Molecular Biology (IUBMB)) include oxidoreductases within this group.

Examples include oxidoreductases selected from those classified under the Enzyme Classification (E.C.) numbers:
Glycerol-3-phosphate dehydrogenase _NAD+_ (1.1.1.8), Glycerol-3-phosphate dehydrogenase _NAO(P)+_ (1.1.1.94), Glycerol-3-phosphate 1-dehydrogenase _NADP_ (1.1.1.94), Glucose oxidase (1.1.3.4), Hexose oxidase (1.1.3.5), Catechol oxidase (1.1.3.14), Bilirubin oxidase (1.3.3.5), Alanine dehydrogenase (1.4.1.1), Glutamate dehydrogenase (1.4.1.2), Glutamate dehydrogenase _NAD(P)+_ (1.4.1.3), Glutamate dehydrogenase_NADP+_ (1.4.1.4), L-Amino acid dehydrogenase (1.4.1.5), Serine dehydrogenase (1.4.1.7), Valine dehydrogenase _NADP+_ (1.4.1.8), Leucine dehydrogenase (1.4.1.9), Glycine dehydrogenase (1.4.1.10), L-Amino-acid oxidase (1.4.3.2.), D-Amino-acid oxidase (1.9.3.3), L-Glutamate oxidase (1.4.3.11), Protein-lysine 6-oxidase (1.4.3.13), L-lysine oxidase (1.4.3.14), L-Aspartate oxidase (1.4.3.16), D-amino-acid dehydrogenase (1.4.99.1), Protein disulfide reductase (1.6.4.4), Thioredoxin reductase (1.6.4.5), Protein disulfide reductase (glutathione) (1.8.4.2), Laccase (1.10.3.2), Catalase (1.11.1.6), Peroxidase (1.11.1.7), Lipoxygenase (1.13.11.12), Superoxide dismutase (1.15.1.1)

Said Glucose oxidases may be derived from Aspergillus niger.

Said Laccases may be derived from Polyporus pinsitus, Myceliophtora thermophila, Coprinus cinereus, Rhizoctonia solani, Rhizoctonia praticola, Scytalidium thermophilum and Rhus vernicifera.

Bilirubin oxidases may be derived from Myrothechecium verrucaria.

The Peroxidase may be derived from e.g. Soy bean, Horseradish or Coprinus cinereus.

The Protein Disulfide reductase may be any mentioned in any of the DK patent applications No. 768/93, 265/94 and 264/94 (Novo Nordisk A/S), which are hereby incorporated as reference, including Protein Disulfide reductases of bovine origin, Protein Disulfide reductases derived from Aspergillus oryzae or Aspergillus niger, and DsbA or DsbC derived from Escherichia coli.

Specific examples of readily available commercial oxidoreductases include Gluzyme^{™} (enzyme available from Novo Nordisk A/S). However, other oxidoreductases are available from others.

It is to be understood that variants of oxidoreductases too are contemplated as the parent enzyme.

The activity of oxidoreductases can be determined as described in "Methods of Enzymatic Analysis", third edition, 1984, Verlag Chemie, Weinheim, vol. 3.

### Parent Carbohydrases

Parent carbohydrases may be defined as all enzymes capable of hydrolyzingcarbohydrate chains (e.g. starches) of particularly five and six member ring structures (i.e. enzymes classified under the Enzyme Classification number E.C. 3.2 (glycosidases) in accordance with the Recommendations (1992) of the International Union of Biochemistry and Molecular Biology (IUBMB)). Also included in the group of carbohydrases according to the invention are enzymes capable of isomerizing carbohydrates e.g. six member ring structures, such as D-glucose to e.g. five member ring structures like D-fructose.

Examples include carbohydrases selected from those classified under the Enzyme Classification (E.C.) numbers:
α-amylase (3.2.1.1) β-amylase (3.2.1.2), glucan 1,4-α-glucosidase (3.2.1.3), cellulase (3.2.1.4), endo-1,3(4)-β-glucanase (3.2.1.6), endo-1, 4-β-xylanase (3.2.1.8), dextranase (3.2.1.11), chitinase (3.2.1.14), polygalacturonase (3.2.1.15), lysozyme (3.2.1.17), β-glucosidase (3.2.1.21), α-galactosidase (3.2.1.22), β-galactosidase (3.2.1.23), amylo-1,6-glucosidase (3.2.1.33), xylan 1,4-β-xylosidase (3.2.1.37), glucan endo-1,3-β-D-glucosidase (3.2.1.39), α-dextrin endo-1,6-glucosidase (3.2.1.41), sucrose α-glucosidase (3.2.1.48), glucan endo-1,3-α-glucosidase (3.2.1.59), glucan 1,4-β-glucosidase (3.2.1.74), glucan endo-1,6-β-glucosidase (3.2.1.75), arabinan endo-1,5-α-arabinosidase (3.2.1.99), lactase (3.2.1.108), chitonanase (3.2.1.132) and xylose isomerase (5.3.1.5).

Examples of relevant carbohydrases include α-1,3-glucanases derived from Trichoderma harzianum; α-1,6-glucanases derived from a strain of Paecilomyces; β-glucanases derived from Bacillus subtilis; β-glucanases derived from Humicola insolens; β-glucanases derived from Aspergillus niger; β-glucanases derived from a strain of Trichoderma; β-glucanases derived from a strain of Oerskovia xanthineolytica; exo-1,4-α-D-glucosidases (glucoamylases) derived from Aspergillus niger; α-amylases derived from Bacillus subtilis; α-amylases derived from Bacillus amyloliquefaciens; α-amylases derived from Bacillus stearothermophilus; α-amylases derived from Aspergillus oryzae; α-amylases derived from non-pathogenic microorganisms; α-galactosidases derived from Aspergillus niger; Pentosanases, xylanases, cellobiases, cellulases, hemi-cellulases deriver from Humicola insolens; cellulases derived from Trichoderma reesei; cellulases derived from non-pathogenic mold; pectinases, cellulases, arabinases, hemi-celluloses derived from Aspergillus niger; dextranases derived from Penicillium lilacinum; endo-glucanase derived from non-pathogenic mold; pullulanases derived from Bacillus acidopullyticus; β-galactosidases derived from Kluyveromyces fragilis; xylanases derived from Trichoderma reesei;

Specific examples of readily available commercial carbohydrases include Alpha-Gal^{™}, Bio-Feed^{™} Alpha, Bio-Feed^{™} Beta, Bio-Feed^{™} Plus, Bio-Feed™ Plus, Novozyme® 188, Carezyme®, Celluclast®, Cellusoft®, Ceremyl®, Citrozym^{™}, Denimax^{™}, Dezyme^{™}, Dextrozyme^{™}, Finizym®, Fungamyl^{™}, Gamanase^{™}, Glucanex®, Lactozym®, Maltogenase^{™}, Pentopan^{™}, Pectinex^{™}, Promozyme®, Pulpzyme^{™}, Novamyl^{™}, Termamyl®, AMG (Amyloglucosidase Novo), Maltogenase®, Sweetzyme®, Aquazym®, Natalase® (all enzymes available from Novo Nordisk A/S). Other carbohydrases are available from other companies.

Carbohydrase variants may function as the parent enzyme.

The activity of carbohydrases can be determined as described in "Methods of Enzymatic Analysis", third edition, 1984, Verlag Chemie, Weinheim, vol. 4.

### Parent Transferases

Parent transferases (i.e. enzymes classified under the Enzyme Classification number E.C. 2 in accordance with the Recommendations (1992) of the International Union of Biochemistry and Molecular Biology (IUBMB)) include transferases within this group.

The parent transferases may be any transferase in the subgroups of transferases: transferases transferring one-carbon groups (E.C. 2.1); transferases transferring aldehyde or residues (E.C 2.2); acyltransferases (E.C. 2.3); glucosyltransferases (E.C. 2.4); transferases transferring alkyl or aryl groups, other that methyl groups (E.C. 2.5); transferases transferring nitrogeneous groups (2.6).
In a preferred embodiment the parent transferase is a transglutaminase E.C 2.3.2.13 (Protein-glutamine µ-glutamyltransferase).

Transglutaminases are enzymes capable of catalyzing an acyl transfer reaction in which a gamma-carboxyamide group of a peptide-bound glutamine residue is the acyl donor. Primary amino groups in a variety of compounds may function as acyl acceptors with the subsequent formation of monosubstituted gamma-amides of peptide-bound glutamic acid. When the epsilon-amino group of a lysine residue in a peptide-chain serves as the acyl acceptor, the transferases form intramolecular or intermolecular gamma-glutamyl-epsilon-lysyl crosslinks.

Examples of transglutaminases are described in the pending DK patent application No. 990/94 (Novo Nordisk A/S).

The parent transglutaminase may be of human, animal (e.g. bovine) or microbial origin.

Examples of such parent transglutaminases are animal derived Transglutaminase, FXIIIa; microbial transglutaminases derived from Physarum polycephalum (Klein et al., Journal of Bacteriology, Vol. 174, p. 2599-2605); transglutaminases derived from Streptomyces sp., including Streptomyces lavendulae, Streptomyces lydicus (former Streptomyces libani) and Streptoverticillium sp., including Streptoverticillium mobaraense, Streptoverticillium cinnamoneum, and Streptoverticillium griseocarneum (Motoki et al., US 5,156,956; Andou et al., US 5,252,469; Kaempfer et al., Journal of General Microbiology, Vol. 137, p. 1831-1892; Ochi et al., International Journal of Sytematic Bacteriology, Vol. 44, p. 285-292; Andou et al., US 5,252,469; Williams et al., Journal of General Microbiology, Vol. 129, p. 1743-1813).

The parent enzyme may also be transferase variants.

The activity of transglutaminases can be determined as described in "Methods of Enzymatic Analysis", third edition, 1984, Verlag Chemie, Weinheim, vol. 1-10.

### Parent Phytases

Parent phytases are included in the group of enzymes classified under the Enzyme Classification number E.C. 3.1.3 (Phosphoric Monoester Hydrolases) in accordance with the Recommendations (1992) of the International Union of Biochemistry and Molecular Biology (IUBMB)).

Phytases are enzymes produced by microorganisms which catalyse the conversion of phytate to inositol and inorganic phosphorus Phytase producing microorganisms comprise bacteria such as Bacillus subtilis, Bacillus natto and Pseudomonas; yeasts such as Saccharomyces cerevisiae; and fungi such as Aspergillus niger, Aspergillus ficuum, Aspergillus awamori, Aspergillus oryzae, Aspergillus terreus or Aspergillus nidulans, and various other Aspergillus species).

Examples of parent phytases include phytases selected from those classified under the Enzyme Classification (E.C.)' numbers: 3-phytase (3.1.3.8) and 6-phytase (3.1.3.26).

The activity of phytases can be determined as described in "Methods of Enzymatic Analysis", third edition, 1984, Verlag Chemie, Weinheim, vol. 1-10, or may be measured according to the method described in EP-A1-0 420 358, Example 2 A.

A composition may comprise at least one protein (polypeptide) or enzyme produced by the method of the invention. The composition may comprise other polypeptides, proteins or enzymes and/or ingredients normally used in personal care products as shampoo, soap bars, skin lotion, skin creme, hair dye, toothpaste, household articles, agrochemicals, personal care products, such as cleaning preparations e.g. for contact lenses, cosmetics, toiletries, oral and dermal pharmaceuticals, compositions used for treating textiles, compositions used for manufacturing food, e.g. baking, and feed etc.

Examples of said proteins(polypeptides)/enzymes include enzymes exhibiting protease, lipase, oxidoreductase, carbohydrase, transferase, such as transglutaminase, phytase and/or anti-microbial polypeptide activity. These enzymes may be present as conjugates with reduced activity.

The protein produced according to the method of the invention may furthermore typically be used in a detergent composition. It may be included in the detergent composition in the form of a non-dusting granulate, a stabilized liquid, or a protected enzyme. Non-dusting granulates may be produced, e.g., as disclosed in US 4,106,991 and 4, 661, 452 (both Novo Industri A/S) and may optionally be coated by methods known in the art. Examples of waxy coating materials are poly(ethylene oxide) products (polyethylene glycol, PEG) with mean molecular weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in patent GB 1483591. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in EP 238,216.

The detergent composition may be in any convenient form, e.g. as powder, granules, paste or liquid. A liquid detergent may be aqueous, typically containing up to 70% water and 0-30 % organic solvent, or non-aqueous.

The detergent composition comprises one or more surfactants, each of which may be anionic, nonionic, cationic, or zwitterionic. The detergent will usually contain 0-50 % of anionic surfactant such as linear alkylbenzenesulfonate (LAS), alpha-olefinsulfonate (AOS), alkyl sulfate (fatty alcohol sulfate) (AS), alcohol ethoxysulfate (AEOS or AES), secondary alkanesulfonates (SAS), alpha-sulfo fatty acid methyl esters, alkyl- or alkenylsuccinic acid, or soap. It may also contain 0-40% of nonionic surfactant such as alcohol ethoxylate (AEO or AE), carboxylated alcohol ethoxylates, nonylphenol ethoxylate, alkylpolyglycoside, alkyldimethylamine oxide, ethoxylated fatty acid monoethanolamide, fatty acid monoethanolamide, or polyhydroxy alkyl fatty acid amide (e.g. as described in WO 92/06154).

The detergent composition may additionally comprise one or more other enzymes, such as e.g. proteases, amylases, lipases, cutinases, cellulases, peroxidases, oxidases, and further anti-microbial polypeptides.

The detergent may contain 1-65 % of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (e.g. SKS-6 from Hoechst). The detergent may also be unbuilt, i.e. essentially free of detergent builder.

The detergent may comprise one or more polymers. Examples are carboxymethylcellulose (CMC), poly(vinylpyrrolidone) (PVP), polyethyleneglycol (PEG), poly (vinyl alcohol) (PVA), polycarboxylates such as polyacrylates, maleic/acrylic acid copolymers and lauryl methacrylate/acrylic acid copolymers.

The detergent may contain a bleaching system which may comprise a H2O2 source such as perborate or percarbonate which may be combined with a peracid-forming bleach activator such as tetraacetylethylenediamine (TAED) or nonanoyloxybenzenesulfonate (NOBS). Alternatively, the bleaching system may comprise peroxyacids of, e.g., the amide, imide, or sulfone type.

The detergent composition comprising a polypeptide produced according to the invention may be stabilized using conventional stabilizing agents, e.g. a polyol such as propylene glycol or glycerol, a sugar or sugar alcohol, lactic acid, boric acid, or a boric acid derivative such as, e.g., an aromatic borate ester, and the composition may be formulated as described in, e.g., WO 92/19709 and WO 92/19708.

The detergent may also contain other conventional detergent ingredients such as, e.g., fabric conditioners including clays, foam boosters, sud suppressors, anti-corrosion agents, soil-suspending agents, anti-soil-redeposition agents, dyes, bactericides, optical brighteners, or perfume.
The pH (measured in aqueous solution at use concentration) will usually be neutral or alkaline, e.g. in the range of 7-11.

### Dishwashing composition

Further, a modified enzyme produced according to the invention may also be used in dishwashing detergents.

Dishwashing detergent compositions comprise a surfactant which may be anionic, non-ionic, cationic, amphoteric or a mixture of these types. The detergent will contain 0-90 % of non-ionic surfactant such as low- to non-foaming ethoxylated propoxylated straight-chain alcohols.

The detergent composition may contain detergent builder salts of inorganic and/or organic types. The detergent builders may be subdivided into phosphorus-containing and non-phosphorus-containing types. The detergent composition usually contains 1-90 % of detergent builders.

Examples of phosphorus-containing inorganic alkaline detergent builders, when present, include the water-soluble salts especially alkali metal pyrophosphates, orthophosphates, and polyphosphates. An example of phosphorus-containing organic alkaline detergent builder, when present, includes the water-soluble salts of phosphonates.

Examples of non-phosphorus-containing inorganic builders, when present, include water-soluble alkali metal carbonates, borates and silicates as well as the various types of water-insoluble crystalline or amorphous alumino silicates of which zeolites are the best-known representatives.

Examples of suitable organic builders include the alkali metal, ammonium and substituted ammonium, citrates, succinates, malonates, fatty acid sulphonates, carboxymetoxy succinates, ammonium polyacetates, carboxylates, polycarboxylates, aminopolycarboxylates, polyacetyl carboxylates and polyhydroxsulphonates.

Other suitable organic builders include the higher molecular weight polymers and co-polymers known to have builder properties, for example appropriate polyacrylic acid, polymaleic and polyacrylic/polymaleic acid copolymers, and their salts.

The dishwashing detergent composition may contain bleaching agents of the chlorine/bromine-type or the oxygen-type. Examples of inorganic chlorine/bromine-type bleaches are lithium, sodium or calcium hypochlorite and hypobromite as well as chlorinated trisodium phosphate. Examples of organic chlorine/bromine-type bleaches are heterocyclic N-bromo and N-chloro imides such as trichloroisocyanuric, tribromoisocyanuric, dibromoisocyanuric and dichloroisocyanuric acids, and salts thereof with water-solubilizing cations such as potassium and sodium. Hydantoin compounds are also suitable.

The oxygen bleaches are preferred, for example in the form of an inorganic persalt, preferably with a bleach precursor or as a peroxy acid compound. Typical examples of suitable peroxy bleach compounds are alkali metal perborates, both tetrahydrates and monohydrates, alkali metal percarbonates, persilicates and perphosphates. Preferred activator materials are TAED and glycerol triacetate.

The dishwashing detergent composition may be stabilized using conventional stabilizing agents for the enzyme(s), e.g. a polyol such as e.g. propylene glycol, a sugar or a sugar alcohol, lactic acid, boric acid; or a boric acid derivative, e.g. an aromatic borate ester.

The dishwashing detergent composition may also contain other conventional detergent ingredients, e.g. deflocculant material, filler material, foam depressors, anti-corrosion agents, soil-suspending agents, sequestering agents, anti-soil redeposition agents, dehydrating agents, dyes, bactericides, fluorescers, thickeners and perfumes.

Finally, the enzyme produced according to the invention may be used in conventional dishwashing detergents, e.g. in any of the detergents described in any of the following patent publications:
EP 518719, EP 518720, EP 518721, EP 516553, EP 516554, EP 5165-55, GB 2200132, DE 3741617, DE 3727911, DE 4212166, DE 4137470, DE 3833047, WO 93/17089, DE 4205071, WO 52/09680, WO 93/18129, WO 93/09153, WO 92/06157, WO 92/08777, EP 429124, WO 93/21299, US 5141664, EP 561452, EP 561446, GB 2234980, WO 93/03129, EP 481547, EP 530870, EP 533239, EP 554943, EP 346137, US 5112518, EP 318204, EP 318279, EP 271155, EP 271156, EP 346136, GB 2.22-8945, CA 2006687, WO 93/25651, EP 530635, EP 414197, US 524063-2.

### Personal care applications:

The protein produced according to the invention is also of interest in connection with personal care applications.

### Proteases

Proteases are well-known active ingredients for the cleaning of contact lenses. They hydrolyse the proteinaceous soil on the lenses and thereby makes it soluble. Removal of the protein soil is essential for the wearing comfort.

Proteases are also effective ingredients in skin cleaning products, where they remove the upper layer of dead keratinaseous skin cells and thereby make the skin look brighter and more fresh.

Proteases are also used in oral care products, especially for cleaning of dentures, but also in dentifrices.

Further, proteases may be used in toiletries, bath and shower products, including shampoos, conditioners, lotions, creams, soap bars, toilet soaps, and liquid soaps.

### Lipases

Lipases can be applied for cosmetic use as active ingredients in skin cleaning products and anti-acne products for removal of excessive skin lipids, and in bath and shower products such as creams and lotions as active ingredients for skin care.

Lipases can also be used in hair cleaning products (e.g. shampoos) for effective removal of sebum and other fatty material from the surface of hair.

Lipases are also effective ingredients in products for the cleaning of contact lenses, where they remove lipid deposits from the lens surface.

### Oxidoreductases

The most common oxidoreductase for personal care purposes is an oxidase (usually glucose oxidase) with substrate (e.g. glucose) that ensures production of H2O2, which then will initiate the oxidation of for instance SCN- or I- into antimicrobial reagents (SCNO- or I2) by a peroxidase (usually lactoperoxidase). In nature enzymatic complex is known from e.g. milk and saliva.

It is being utilised commercially as anti-microbial system in oral care products (mouth rinse, dentifrice, chewing gum) where it also can be combined with an amyloglucosidase to produce the glucose. These systems are also known in cosmetic products for preservation.

Anti-microbial systems comprising the combination of an oxidase and a peroxidase are known in the cleaning of contact lenses.

Another application of oxidoreductases is oxidative hair dyeing using oxidases, peroxidases and laccases.

Free radicals formed on the surface of the skin (and hair) are known to be associated with the ageing process of the skin. The free radicals activate chain reactions that lead to destruction of fatty membranes, collagen, and cells. The application of free radical scavengers such as Superoxide dismutase into cosmetics is well-known (R. L. Goldemberg, DCI, Nov. 93, p. 48-52).

Protein disulfide isomerase (PDI) is also an oxidoreductase. It can be utilised for waving of hair (reduction and reoxidation of disulfide bonds in hair) and repair of spoiled hair (where the damage is mainly reduction of existing disulfide bonds).

### Carbohydrases

Plaque formed on the surface of teeth is composed mainly of polysaccharides. They stick to the surface of the teeth and the microorganisms. The polysaccharides are mainly α-1,6 bound glucose (dextran) and α-1,3 bound glucose (mutan). The application of different types of glucanases such as mutanase and dextranase helps hydrolysing the sticky matrix of plaque, making it easier to remove by mechanical action.

Also other kinds of biofilm for instance the biofilm formed in lens cases can be removed by the action of glucanases.

### Food and Feed

Further conjugated enzymes or polypeptides with reduced allergenicity produced according to the invention may advantageously be used in the manufacture of food and feed.

### Proteases

The gluten in wheat flour is the essential ingredient responsible for the ability of flour to be used in baked foodstuffs. Proteolytic enzymes are sometimes needed to modify the gluten phase of the dough, e.g. a hard wheat flour can be softened with a protease.

Neutrase® is a commercially available neutral metallo protease that can be used to ensure a uniform dough quality and bread texture, and to improve flavour. The gluten proteins are degraded either moderately or more extensively into peptides, whereby close control is necessary in order to avoid excessive softening of the dough.

Proteases are also used for modifying milk protein. To coagulate casein in milk when producing cheese proteases such as rennet or chymosin may be used.

In the brewery industry proteases are used for brewing with unmalted cereals and for controlling the nitrogen content.
In animal feed products proteases are used so to speak to expand the animals digestion system.

### Lipases

The application of lipase in the baking industry is rather new. Addition of lipase results in improved dough properties and an improved bread making quality in terms of larger volume, improved crumb structure and whiter crumb colour. The observed effect can be explained by a mechanism where the lipase changes the interaction between gluten and some lipids fragment during dough mixing. This results in an improved gluten network.

The flavour development of blue roan cheeses (e.g. Danablue), certain Italian type cheese and other dairy products containing butter fat are dependent on the degradation of milk fat into free fatty acids. Lipases may be used for developing flavour in such products.

In the oil- and fat producing industry lipases are used e.g. to minimise the amount of undesirable side-products, to modify fats by interesterification, and for the synthesis of esters.

### Oxidoreductases

Further oxidoreductases with reduced allergenicity produced according to the invention may advantageously be used in the manufacture of food and feed.

Several oxidoreductases are used for baking, glucose oxidase, lipoxygenase, peroxidase, catalase and combinations hereof. Traditionally, bakers strengthen gluten by adding ascorbic acid and potassium bromate. Some oxidoreductases can be used to replace bromate in dough systems by oxidation of free sulfydryl units in gluten proteins. Hereby disulphide linkages are formed resulting in stronger, more elastic doughs with greater resistance .

Gluzyme^{™} (Novo Nordisk A/S) is a glucose oxidase preparation with catalase activity that can be used to replace bromate. The dough strengthen is measured as greater resistance to mechanical shock, better oven spring and larger loaf volume.

### Carbohydrases

Flour has varying content of amylases leading to differences in the baking quality. Addition of amylases can be necessary in order to standardise the flour. Amylases and pentosanases generally provide sugar for the yeast fermentation, improve the bread volume, retard retrogradation, and decrease the staling rate and stickiness that results from pentosan gums. Examples of carbohydrases are given below.

Certain maltogenic amylases can be used for prolonging the shelf life of bread for two or more days without causing gumminess in the product. The mechanism behind this is that the amylases selectively modify the gelatinized starch by the cleavage from the non-reducing end of the starch molecules, of low molecular weight sugars and dextrins. The starch is modified in such a way that retrogradation is less likely to occur. The produced low-molecular-weight sugars improve the baked goods water retention capacity without creating the intermediate-length dextrins that result in gumminess in the finished product. The enzyme is inactivated during bread baking, so it can be considered a processing aid that does not have to be declared on the label. Overdosing of Novamyl can almost be excluded.

The bread volume can be improved by fungal α-amylases which further provide a good and uniform structure of the bread crumb. Said α-amylases are endoenzymes that produce maltose, dextrins and glucose. Cereal and some bacterial α-amylases are inactivated at temperatures above the gelatinisation temperature of starch, therefore when added to wheat dough a low bread volume and a sticky bread interior are achieved. Fungamyl has the advantage of being thermolabile and is inactivated just below the gelatinisation temperature.

Enzyme preparations containing a number of pentosanase and hemi-cellulase activities can improve the handling and stability of the dough, and improves the freshness, the crumb structure and the volume of the bread.

By hydrolysing the pentosans fraction in flour it will lose a great deal of its water-binding capacity, and the water will then be available for starch and gluten. The gluten becomes more pliable and extensible, and the starch will gelatinise more easily. Pentosanases can be used in combination with or as an alternative to emulsifiers.

Further carbohydrases are used for producing syrups from starch, which are widely used in soft drinks, sweets, meat products, dairy products, bread products, ice cream, baby food, jam etc.

The conversion of starch is normally carried out in three steps. First the starch is liquefied, by the use of α-amylases. Maltodextrins, primary consisting of oligosaccharides and dextrins, are obtained.

The mixture is then treated with an amyloglucosidase for hydrolysing the oligosaccharides and dextrins into glucose. This way a sweeter product is obtained. If high maltose syrups are desired β-amylases alone or in combination with a pullulanase (de-branching enzyme) may be used.

The glucose mixture can be made even sweeter by isomerisation to fructose. For this purpose an immobilized glucose isomerase can be used.

In the sugar industry, it is common practice to speed up the break down of starch in cane juices. Thereby the starch content in the raw sugar is reduced and filtration at the refinery facilitated.

Furthermore dextranases are used to break down dextran in raw sugar juices and syrups.

In the alcohol industry α-amylases are advantageously being used for the thinning of starch in distilling mashes.

In the brewing industry α-amylases are used for adjunct liquefaction.

In the dairy industry β-galactosidases (lactases) are used when producing low lactose milk for persons suffering from lactose malabsorption.

When flavoured milk drinks are produced from lactase-treated milk, the addition of sugar can be reduced without reducing the sweetness of the product.

In the production of condensed milk, lactose crystallisation can be avoided by lactase treatment, and the risk of thickening caused by casein coagulation in lactose crystals is thus reduced.

When producing ice cream made from lactase-treated milk (or whey) no lactose crystals will be formed and the defect sandiness will not occur.

Further, xylanases are known to be used within a number of food/feed industrial applications as described in WO 94/21785 (Novo Nordisk A/S).

α-amylases are used in the animal feed industry to be added to cereal-containing feed to improve the digestibility of starch.

### Anti-microbial polypeptides

Certain bacteriolytic enzymes may be used e.g. to wash carcasses in the meat packing industry (see US patent No. 5,354,681 from Novo Industri A/S)

### Transferases

Transglutaminases with reduced allergenicity produced according to the invention may advantageously be used in the manufacturing of food and feed.

Transglutaminases have the ability of cross-linking proteins. This property can be used for the gelling of aqueous phases containing proteins. This may be used when producing spreads (DK patent application No. 1071/84 from Novo Nordisk A/S).

Transglutaminases are being used for the improvement of the baking quality of flour e.g. by modifying wheat flour to be used in the preparation of cakes with improved properties, such as improved taste, dent, mouth-feel and a higher volume (see JP 1-110147).

Further producing paste type food material e.g. used as fat substitution in foods as ice cream, toppings, frozen desserts, mayonnaise and low fat spreads (see WO 93/22930 from Novo Nordisk A/S).

Furthermore for preparation of gels for yoghurt, mousses, cheese, puddings, orange juice, from milk and milk-like products, and binding of chopped meat product, improvement of taste and texture of food proteins (see WO 94/21120 and WO 94/21129 from Novo Nordisk A/S).

### Phytases

Phytases produced according to the method of the invention may advantageously be used in the manufacturing of food, such as breakfast cereal, cake, sweets, drink, bread or soup etc., and animal feed.

Phytases may be used either for exploiting the phosphorus bound in the phytate/phytic acid present in vegetable protein sources or for exploiting the nutritionally important minerals bound in phytic acid complexes.

Microbial phytase may be added to feedstuff of monogastric animals in order to avoid supplementing the feed with inorganic phosphorus (see US patent No. 3,297,548)

Further phytases may be used in soy processing. Soybean meals may contain high levels of the anti-nutritional factor phytate which renders this protein source unsuitable for application in baby food and feed for fish, calves and other non-ruminants, since the phytate chelates essential minerals present therein. (see EP 0 420 358).

Also for baking purposes phytases may be used. Bread with better quality can be prepared by baking divided pieces of a dough containing wheat flour etc. and phytase (see JP-0-3076529-A)

A high phytase activity in koji mold is known to be used for producing refined sake (see JP-0-6070749-A).

### Textile applications:

### Proteases

Proteases are used for degumming and sand-washing of silk.

### Lipases

Lipases are used for removing fatty matter containing hydrophobic esters (e.g. triglycerides) during the finishing of textiles (see e.g. WO 93/13256 from Novo Nordisk A/S).

### Oxidoreductases

In bleach clean-up of textiles catalases may serve to remove excess hydrogen peroxide.

### Carbohydrases

Cellulolytic enzymes are widely used in the finishing of denim garments in order to provide a localized variation in the colour density of the fabric (Enzyme facilitated "stone wash").

Also cellulolytic enzymes find use in the bio-polishing process. Bio-Polishing is a specific treatment of the yarn surface which improves fabric quality with respect to handle and appearance without loss of fabric wettability. Bio-polishing may be obtained by applying the method described e.g. in WO 93/20278.

During the weaving of textiles, the threads are exposed to considerable mechanical strain. In order to prevent breaking, the threads are usually reinforced by the coating (sizing) of a gelatinous substance (size). The most common sizing agent is starch in native or modified form. A uniform and durable finishing can thus be obtained only after removal of the size from the fabric, the so called desizing. Desizing of fabrics sized with a size containing starch or modified starch is preferably facilitated by the use of amylolytic enzymes.

### Oral and dermal pharmaceuticals:

### Proteases

Different combinations of highly purified proteases (e.g. Trypsin and Chymotrypsin) are used in pharmaceuticals to be taken orally, and dermal pharmaceuticals for combating e.g inflammations, edemata and injuries.

### Leather production:

### Transferase

Transglutaminase is known for the use in casein finishing of leather by acting as a hardening agent (see WO 94/13839 from Novo Nordisk).

### Hard surface cleaning:

Cleaning of hard surfaces e.g. in the food industry is often difficult, as equipment used for producing dairies, meat, sea food products, beverages etc. often have a complicated shape. The use of surfactant compositions in the form gels and foams comprising enzymes have shown to facilitate and improve hard surface cleaning. Enzymes, which advantageously may be added to such surfactant compositions, are in particular proteases, lipases, amylases and cellulases.

Such hard surface cleaning compositions comprising enzymes may also advantageously be used in the transport sector, for instance for washing cars and for general vessel wash.

Finally the invention relates to the use of the protein produced according to the method of the invention or a composition comprising the protein in products comprising polypeptides.

First of all the conjugate or composition produced according to the invention can advantageously be used for personal care products, such as hair care and hair treatment products. This include products such as shampoo, hair conditioners, hair waving compositions, hair dyeing compositions, hair tonic, hair liquid, hair cream, hair rinse, hair spray.

Further contemplated are oral care products such as dentifrice, mouth rinse, chewing gum.

Additionally contemplated are skin care products like cosmetics, such as skin cream, skin milk, cleansing cream, cleansing lotion, cleansing milk, cold cream, cream soap, nourishing essence, skin lotion, milky lotion, calamine lotion, hand cream, powder soap, transparent soap, sun oil, sun screen, shaving foam, shaving cream, baby oil lipstick, lip cream, creamy foundation, face powder, powder eye-shadow, powder, foundation, make-up base, essence powder, whitening powder.

Also for contact lenses hygiene products the conjugate of the invention can be used advantageously. Such products include contact lenses cleansing and disinfection products.

The use of detergents such as washing powder, soap, soap bars, liquid soap are also contemplated.

The invention may involve the use of a DNA construct encoding a glycosylated protein variant and an expression vector comprising the DNA construct. The expression vector may be any vector subjected to the procedure of recombinant DNA, followed by successful expression in a host organism. When introduced into the host organism the vector may function in an autonome fashion. By a replication origin the plasmid can replicate outside the host cell genome. Alternatively it can be incorporated into the host cell genome and be replicated together with the host cell genome.

Furthermore the present invention can involve a process by which a glycosylated protein variant is being synthesised and expressed. The steps toward creating the final product are achieved by cultivating a host capable of glycosylating and expressing a protein in a suitable medium to obtain expression and secretion of the glycosylated protein variant into the medium. Following this is the recovery and isolation of the protein from the culture medium.

Another aspect of the present invention is the production of protein variants in a host cell. The host cell may be any cell suitable for the large-scale production of proteins, capable of expressing a protein and being transformed by an expression vector. A further aspect of the present invention is a host capable of glycosylation. The host may be selected from bacterial, fungal, animal cells or transgenic plant cells. The animal cells may be insect or mammalian cells, for example.

In one embodiment the host is yeast. The yeast cell may be selected from the groups consisting of Saccharomyces cerevisiae, Saccharomyces uvae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichummn sp., and Geotrichum fermentans.

In another embodiment the host is selected among other fungi, such as Humicola lanuginosa or Aspergillus oryzae.

In yet another embodiment the host is selected among plant cells, such as transgenic plant cells, for example potatoe cells, or the host is selected among animal cells, such as insect cells, for example Spodoptera frugiperda Sf9 and Sf21 ovary cells , or mammalian cells, such as monkey CV1 and COS cells, murine C127 fibroblasts and chinese hamster ovary cells (CHO).

The selected host is cultured in a suitable medium, whereby said protein variant is expressed and glycosylated in the host. Finally the glycosylated protein variant is recovered from the medium.

By making a specific choice of host it is possible to select the nature of glycosylation of the glycosylated protein variant.

### Materials and methods

### Materials

### Enzymes:

Lipolase: Lipase derived from Thermomyces lanuginosus (Humicola lanuginosa)
strain DSM 4109 by means of genetic engineering.

### Materials, chemicals and solutions:

Horse Radish Peroxidase labeled anti-rat-Ig (Dako, DK, P162, # 031; dilution 1:1000).
Mouse anti-rat IgE (Serotec MCA193; dilution 1:200).
Rat anti-mouse IgE (Serotec MCA419; dilution 1:100).
Biotin-labeled mouse anti-rat IgGl monoclonal antibody (Zymed 03-9140; dilution 1:1000)
Biotin-labeled rat anti-mouse IgGl monoclonal antibody (Serotec MCA336B; dilution 1:1000)
Streptavidin-horse radish peroxidase (Kirkegård & Perry 14-30-00; dilution 1:1000).
CovaLink NH₂ plates (Nunc, Cat# 459439)
Cyanuric chloride (Aldrich)
Acetone (Merck)
Rat anti-Mouse IgGl, biotin (SeroTec, Cat# MCA336B)
Streptavidin, peroxidase (KPL)
Ortho-Phenylene-diamine (OPD) (Kem-en-Tec)
H₂O₂, 30% (Merck)
Tween 20 (Merck)
Skim Milk powder (Difco)
H₂SO₄ (Merck)

| Buffers and Solutions: | | | |
|---|---|---|---|
| Carbonate buffer (0.1 M, pH 10 (1 liter)) Na₂CO₃ | | | 10.60 g |
| PBS (pH 7.2 (1 liter)) | NaCl | 8.00 g | |
| | KCl | | 0.20 g |
| | K₂HPO₄ | 1.04 g | |
| | KH₂PO₄ | 0.32 g | |
| Washing buffer PBS, 0.05% (v/v) Tween 20 | | | |
| Blocking bufferPBS, 2% (wt/v) Skim Milk powder | | | |
| Dilution buffer PBS, 0.05% (v/v) Tween 20, 0.5% (wt/v) Skim Milk powder | | | |
| Citrate buffer (0.1M, pH 5.0-5.2 (1 liter))NaCitrate | | | 20.60 g |
| Citric acid | | | 6.30 g |

### Activation of CovaLink plates:

· Make a fresh stock solution of 10 mg cyanuric chloride per ml acetone.
· Just before use, dilute the cyanuric chloride stock solution into PBS, while stirring, to a final concentration of 1mg/ml.
· Add 100 ml of the dilution to each well of the CovaLink NH2 plates, and incubate for 5 minutes at room temperature.
· Wash 3 times with PBS.
· Dry the freshly prepared activated plates at 50°C for 30 minutes.
· Immediately seal each plate with sealing tape.
· Preactivated plates can be stored at room temperature for 3 weeks when kept in a plastic bag.
Sodium Borate, borax (Sigma)
3,3-Dimethyl glutaric acid (Sigma)
CaCl₂ (Sigma)
Tresyl chloride (2,2,2-triflouroethansulfonyl chloride) (Fluka)
1-ethyl-3-(3-dimethylaminopropyl)carbodiimide (EDC) (Fluka)
N-Hydroxy succinimide (Fluka art. 56480))
Phosgene (Fluka art. 79380)
Lactose (Merck 7656)
PMSF (phenyl methyl sulfonyl flouride) from Sigma
Succinyl-Alanine-Alanine-Proline-Phenylalanine-para-nitroanilide (Suc-AAPF-pNP) Sigma no. S-7388, Mw 624.6 g/mole.

### Colouring substrate:

OPD: o-phenylene-diamine, (Kementec cat no. 4260)

### Test Animals:

Female Balb/C mice (about 20 grams) purchased from Bomholdt-gaard, Ry, Denmark.
Female Brown-Norway rats, weighing on the average 180 g

### Equipment:

XCEL II (Novex)
ELISA reader (UVmax, Molecular Devices)
HPLC (Waters)
PFLC (Pharmacia)
Superdex-75 column, Mono-Q, Mono S from Pharmacia, SW.
SLT: Fotometer from SLT LabInstruments
Size-exclusion chromatograph (Spherogel TSK-G2000 SW).
Size-exclusion chromatograph (Superdex 200, Pharmacia, SW)
Amicon Cell

### Enzymes for DNA manipulations

Unless otherwise mentioned all enzymes for DNA manipulations, such as e.g. restriction endonucleases, ligases etc., are obtained from New England Biolabs. Inc.

### Media:

| BPX: Composition (per liter) | |
|---|---|
| Potato starch | 100g |
| Ground barley | 50g |
| Soybean flour | 20g |
| Na₂HPO₄ X 12 H₂O | 9g |
| Pluronic | 0.1g |
| Sodium caseinate | 10g |

The starch in the medium is liquefied with α-amylase and the medium is sterilized by heating at 120°C for 45 minutes. After sterilization the pH of the medium is adjusted to 9 by addition of NaHCO₃ to 0.1 M.

### Methods

### General molecular biology methods:

Unless otherwise mentioned the DNA manipulations and transformations were performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).
Enzymes for DNA manipulations were used according to the specifications of the suppliers.

### Immunisation of mice

Balb/C mice (20 grams) are immunised 10 times (intervals of 14 days) by subcutaneous injection of the modified or unmodified polypeptide in question, respectively by standard procedures known in art.

### Immunisation of rats.

Twenty intratracheal immunisations on rats were performed weekly with 100 µl 0.9 % (wt/vol) NaCl (control group) or 100 µl of the protein dilution mentioned above. Group 1 received wild type lipolase, group 2 received lipolase variant #1, and group 3 received lipolase variant #5. Each group contained 10 rats. Blood samples (2 ml) were collected from the eye a week after every second immunisation. The serum was obtained by blood clothing and centrifugation.

### ELISA IgE test system (for Brown Norway rats):

A three layer sandwich ELISA is used to determine relative concentrations of specific antibodies.

The immunizing molecule is used as coating antigen with 10 mg per ml and 50 ml per well, in neutral phosphate buffer, incubated overnight at 4°C. All remaining binding spots on the well surface are blocked in 2 % skim milk, 200 ml per well in phosphate buffer for at least 30 minutes at room temperature (RT). All seras to be tested with this antigen are added at 50 ml per well to this plate using a 8-channel pipette in dilution series from 10 x diluted followed by 3-fold dilutions. Dilutions are made in phosphate buffer with 0.5 % skim milk and 0.05% Tween20, incubated 2 hours on agitation platform at RT. The "tracer" molecule is biotinylated Mouse anti Rat IgE 50 ml per well and diluted 2000 x in phosphate buffer with 0.5 % skim milk and 0.05% Tween 20, incubated 2 hours on an agitation platform at RT. Control (blank) was identical sequence but without rat sera. 50 ml per well streptavidin horse raddish peroxidase, diluted 2000 x was incubated 1 hour on an agitation platform. Colouring substrate at 50 ml per well is OPD (6 mg) and H₂O₂ (4 ml of a 30% solution) per 10 ml citrate buffer pH 5.2. The reaction is stopped using 100 ml per well 2 N H₂SO₄. All readings on SLT at 486 nm and 620 nm as reference. Data is calculated and presented in Lotus.

### ELISA procedure to determine relative concentrations of IgE antibodies in BALB/C mice

A three layer sandwich ELISA is used to determine relative concentrations of specific IgE serum antibodies.
1) Coat the ELISA-plate with 10 mg rat anti-mouse IgE or mouse anti-rat IgE/ml buffer 1.
   50 ml/well. Incubate over night at 4°C.
2) Empty the plates and block with Blocking buffer at least ½ hour at room temperature.
   200 ml/well. Shake gently. Wash the plates 3 times with Washing Buffer.
3)Incubate with mouse/rat sera, starting from undiluted and continue with 2-fold dilutions. Keep
   some wells free for buffer 4 only (blanks). 50 ml/well. Incubate for 30 minutes at room temperature. Shake gently. Wash the plates 3 times in Washing Buffer.
4) Dilute the enzyme in Dilution buffer to the appropriate protein concentration. 50ml/well.
   Incubate for 30 minutes at room temperature. Shake gently. Wash the plates 3 times in Washing Buffer.
5) Dilute specific polyclonal anti-enzyme antiserum serum (pIg) for detecting bound antibody in Dilution buffer. 50ml/well. Incubate for 30 minutes at room temperature. Shake gently. Wash the plates 3 times in Washing Buffer.
6) Dilute Horseradish Peroxidase-conjugated anti-pIg-antibody in Dilution buffer. 50 ml/well.
   Incubate at room temperature for 30 minutes. Shake gently. Wash the plates 3 times in Washing Buffer.
7) Mix 0.6 mg ODP/ml + 0.4 µl H₂O₂/ml in substrate Buffer. Make the solution just before use. Incubate for 10 minutes. 50 µl/well.
8) To stop the reaction: add Stop Solution. 50 µl/well.
9) Read the plates at 492 nm with 620 nm as reference.
   Data is calculated and presented in Lotus.

### Balb/C mice IgG ELISA Procedure:

· The antigen is diluted to 1 mg/ml in carbonate buffer.
· 100 µl is added to each well.
· The plates are coated overnight at 4°C.
· Unspecific adsorption is blocked by incubating each well for 1 hour at room temperature with 200 µl blocking buffer.
· The plates are washed 3x with 300 µl washing buffer.
· Unknown mouse sera are diluted in dilution buffer, typically 10x, 20x and 40x, or higher.
· 100 ml is added to each well.
· Incubation is for 1 hour at room temperature.
· Unbound material is removed by washing 3x with washing buffer.
· The anti-Mouse IgG1 antibody is diluted 2000x in dilution buffer.
· 100 µl is added to each well.
· Incubation is for 1 hour at room temperature.
· Unbound material is removed by washing 3x with washing buffer.
· Streptavidine is diluted 1000x in dilution buffer.
· 100 µl is added to each well.
· Incubation is for 1 hour at room temperature.
· Unbound material is removed by washing 3x with 300 ml washing buffer.
· OPD (0.6 mg/ml) and H₂O₂ (0.4 ml/ml) is dissolved in citrate buffer.
· 100 µl is added to each well.
· Incubation is for 10 minutes at room temperature.
· The reaction is stopped by adding 100 µl H₂SO₄.
· The plates are read at 492 nm with 620 nm as reference.

### Molecular biological methods:

Site-directed mutagenesis, cloning and expression of enzyme variants in yeast and purification of the variants were performed according to the state of the art technologies.

ELISA detecting antigen-specific rat IgE: This ELISA is a capturing ELISA, and was performed on CovaLink NH2 plates activated with cyanuric chloride as described by the manufacturer. The ELISA was performed in phosphate buffered saline under conditions commonly known. Plates were coated overnight with 100 µl mouse anti-Rat IgE (5 µg/ml). Folowing this stage Tween 20 was added to the buffer solutions to a final concentration of 0.01 % (v/v). Unspecific adsorption was blocked with 2 % (w/v) skim milk. Unknown rat sera were diluted in 0.5 % (w/v) skim milk, typically 10 x, 20 x and 40 x, and were added to the wells. Lipolase was diluted to 1 µg/ml in 0.5 % (w/v) skim milk, and 100 µl was added to each well. Immobilised enzyme was detected with antigen-specific polyclonal serum in 0.5 % (w/v) skim milk. Anti-lipolase immunoglobulins were detected using anti-rabbit immunoglobulin antibodies labelled with horseradish peroxidase.

ELISA detecting antigen-specific rat IgG1: This ELISA is a direct ELISA, and was performed on CovaLink NH2 plates activated with cyanuric chloride as described by the manufacturer. The ELISA was performed in phosphate buffered saline under conditions commonly known. Plates were coated overnight with 100 µl antigen (5 µg/ml).). Following this stage Tween 20 was added to the buffer solutions to a final concentration of 0.01 % (v/v). Unspecific adsorption was blocked with 2 % skim milk. Unknown rat sera were diluted in 0.5 % (w/v) skim milk, typically 10 x, 20 x and 40 x, and were added to the wells. Immobilised antigen-specific rat IgG1 was detected using a biotinylated mouse anti-rat IgG1 in 0.5 % (w/v) skim milk. Immobilised mouse anti-rat IgG1 was detected using a streptavidine-bound horseradish peroxidase.

### Statistical analysis:

Differences between data sets were analysed by using non-parametric methods: the Kruskal-Wallis Test and the Dunn's Multiple Comparison Test.

### Examples

The following experiments illustrate the reduction of allergenicity of the enzyme lipase (E.C.3.1.1.3) from Thermomyces lanuginosus (Humicola lanuginosa) strain DSM 4109 by means of genetic engineering. The enzyme was provided with extra glycosylation sites in specific areas of the enzyme.

### Example 1: Epitope identification and variant selection.

Lipase variants were designed to introduce additional glycosylation sites of the consensus sequence Asn-Xaa-Thr/Ser. First, the epitopes on lipase wild-type were identified, in order to choose glycosylation sites, to maximize the effects of both macrophage scavenging and epitope shielding.

To identify epitope patterns, phage display libraries expressing random peptide sequences (9-mers) were screened with IgG purified from rabbit anti-lipase antisera by caprylic acid precipitation. IgG antibodies were immobilised on paramagnetic microspheres carrying specific anti-rabbit IgG antibodies, as described by the manufacturer (Sigma). Unbound material was removed and the coated microspheres were isolated and incubated with the phage display library expressing 9-mer oligopeptides with random amino acid sequences. The bound phages were eluted from the microspheres and incubated with an appropriate Escheria coli strain in the presence of a carrier-phage and then plated in line with the existing state of the art. Plates that presented plaques were screened for specific binding to anti-lipase antibodies according to the state of the art. For each plate two copies were made on nitrocellulose filters. Filter #1 was incubated with purified lipase-specific IgG while filter #2 was incubated with purified lipase-specific IgG in the presence of excess lipase. Both filters were developed using anti-rabbit IgG labelled with alkaline phosphatase. Plaques appeared on filter #1 but not on filter #2, were selected and cultured according to the state of the art. Reactive oligopeptide sequences were identified by automated DNA sequence analysis, and their sequences aligned to identify 5 important epitopes:
1. R P P R
2. > E Y
3. P > P A P > S
4. > R S A
5. L > G R S

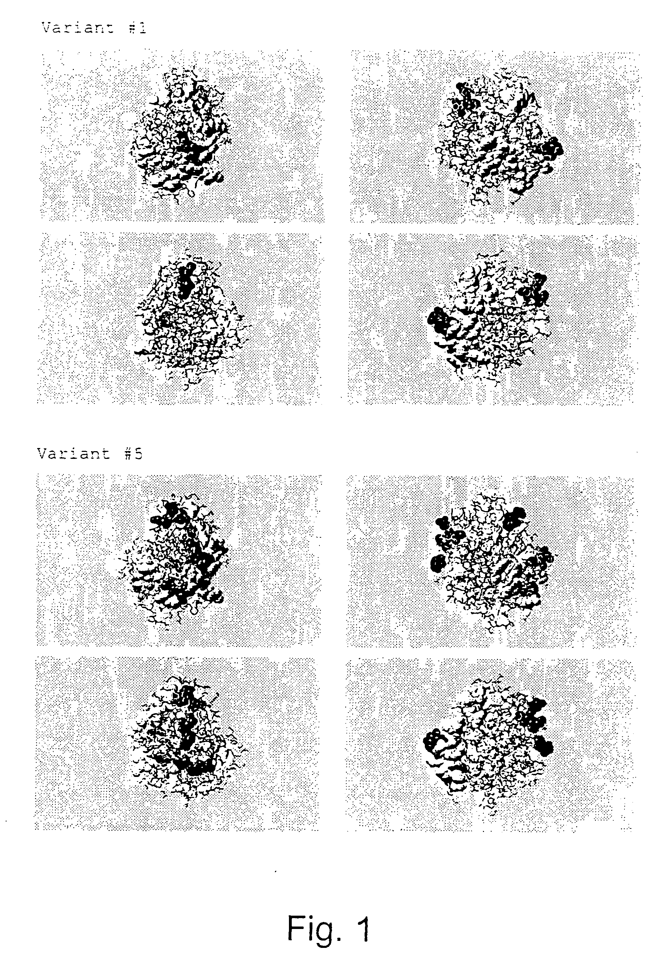

These epitope patterns were localised on the 3D-structure of the lipase molecule (1tib.pdb) by computer analysis. They mapped at the N-terminal extension (lip.1) and to positions around residues E129-Y164 (lip.2.1), E129-Y220 (lip.2.2-4), P253-P250-A243-P208/207-S214/216/217 (lip.3.0), R209-S214-Y220 (lip.4.0), L67-G65-R81-S83/85 (lip.5.1) and L96/97-G212-R209/179-S214 (lip.5.2), respectively (Figure 1).

According to the information obtained from phage display screening, variants having additional glycosylation sites close to these epitopes were constructed by the means of protein engineering. Figure 1 shows two such variants, #1 with one additonal glycosylation site and #5 with 4 additional glycosylation sites. Lipase itself has one glycosylation site (N33 = Asn₃₃-Ile₃₄-Thr₃₅) situated at a distance from the identified epitopes. In variant #1 one epitope is predicted to be affected by the extra glycosylation site, while 3 epitopes are affected by the extra glycosylation in variant #5.

**Table 1: Substitutions introduced to create additional glycosylation sites.**

| Variant | Glycosylati on sites | Correspondi ng substitutions | Epitope affected |
|---|---|---|---|
| wild type | N33 | none | |
| #1 | N33 | | lip.5.2 |
| | N99 | E99N,N101S | |
| #5 | N33 | | lip.2.1 |
| | N37 | T37N,N39S | lip.2.2-4 |
| | N99 | E99N,N101S | lip.3.0 |
| | N163 | G163N,D165S | lip.4.0 |
| | N212 | G212N | lip.5.2 |

### Example 2: Construction and expression of glycosylated variants.

Site-directed mutagenesis and cloning of the enzyme variants were performed by standard techniques well known in the art (see, e.g., Sambrook et al. (1989), Sambrook et al., Molecular Cloning. A Laboratory Manual, Cold Spring Harbor, NY; Ford et al., 1991, Protein Expression and Purification 2, p. 95-107. The gene sequence of the wild type lipase can be found in the EMBL/Genbank database with accession number AF054513, and the vector used was CaHj483 (WO 99/42566). The resulting expression vectors were transformed in Aspergillus oryzae JaL 228 (PCT/DK 97/00037) using selection on acetamide as described in patent EP 531,372 B1. Transformants were spore reisolated fermented in shake-flasks to yield the lipase variants. The resulting lipase variants were recovered by methods known in the art (Svendsen et al., Methods in Enzymology, vol 284, pp317-340, 1997).

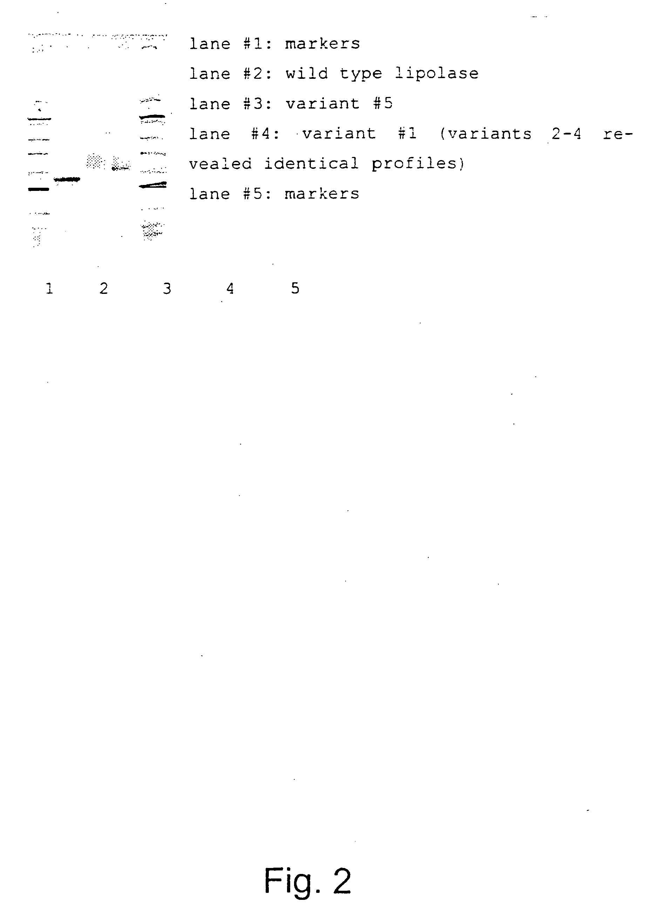

Thus, the lipase variants were expressed in Aspergillus oryzae, which is known to be capable of glycosylation. The secreted lipase variants were glycosylated in the cells as demonstrated by the mobility shift of the variants compared to the wild type in Western blot analysis (Figure 2). Furthermore, the slow migration of the variant (#5) with most glycosylation sites indicates that the extent of glycosylation correlates to the number of glycosylation sites introduced in the sequence, as expected.

### Example 3: Retained functionality of variants

Wild-type lipase and the variants were purified and their specific actvity was determined. Briefly, a substrate for lipase is prepared by emulsifying glycerin tributyrate using gum Arabic as emulsifier. Lipase activity is assayed at pH 7 using pH stat method. One unit of lipase activity (LU) is defined as the amount needed to liberate one micromole fatty acid per minute (Svendsen et al., Methods in Enzymology, vol 284, pp317-340, 1997).

The activity is expressed as LU/ml of solutions with the same protein concentration, determined by A₂₈₀ measurement. Wild-type lipase has an activity of 4917 LU/ml, variant #1 has 4478 LU/ml and variant #5 has 4222 LU/ml. Hence, the variants have essentially the same functionality as the wild-type lipase.

### Example 4: IT-Rat studies (IgGl/IgE):

The antigenic and allergenic potencies of variants #1 and #5 and of wild type lipase were compared in a rat model with intratracheal exposure to antigen.

Immunisation: Twenty intratracheal immunisations on rats (Female Brown-Norway rats with an average weight of 180g) were performed weekly with 100 µl 0.9 % (wt/vol) NaCl (control group) or 100 µl salt solution containing 15 µg of protein. Group 1 received wild type lipase, group 2 received lipase variant #1, and group 3 received lipase variant #5. Each group contained 10 rats. Blood samples (2 ml) were collected from the eye one week after every second immunisation. The serum was obtained by blood clothing and centrifugation.

ELISA detecting antigen-specific rat IgE: This ELISA is a capturing ELISA, and was performed on CovaLink NH2 plates (Nunc, Denmark) activated with cyanuric chloride as described by the manufacturer. The ELISA was performed in phosphate buffered saline under standard conditions. Plates were coated overnight with 100 µl mouse anti-Rat IgE (5 µg/ml). Following this stage, Tween 20 was added to the buffer solutions to a final concentration of 0.01 % (v/v). Unspecific adsorption was blocked with 2 % (w/v) skim milk. Unknown rat sera were diluted in 0.5 % (w/v) skim milk, typically 10 x, 20 x and 40 x, and were added to the wells. Lipase was diluted to 1 µg/ml in 0.5 % (w/v) skim milk, and 100 µl was added to each well. Immobilised enzyme was detected with antigen-specific polyclonal serum in 0.5 % (w/v) skim milk. Anti-lipase immunoglobulins were detected using anti-rabbit immunoglobulin antibodies labelled with horseradish peroxidase.

ELISA detecting antigen-specific rat IgG1: This ELISA is a direct ELISA, and was performed on CovaLink NH2 plates (Nunc, Denmark) activated with cyanuric chloride as described by the manufacturer. The ELISA was performed in phosphate buffered saline under standard conditions. Plates were coated overnight with 100 µl antigen (5 µg/ml). Following this stage, Tween 20 was added to the buffer solutions to a final concentration of 0.01 % (v/v). Unspecific adsorption was blocked with 2 % skim milk. Unknown rat sera were diluted in 0.5 % (w/v) skim milk, typically 10 x, 20 x and 40 x, and were added to the wells. Immobilised antigen-specific rat IgG1 was detected using a biotinylated mouse anti-rat IgG1 in 0.5 % (w/v) skim milk. Immobilised mouse anti-rat IgG1 was detected using a streptavidine-bound horseradish peroxidase.

Differences between data sets were analysed by using non-parametric methods: the Kruskal-Wallis Test and the Dunn's Multiple Comparison Test. The allergenicity is summarized as "the end-point IgE antibody level" and the "integrated IgE antibody response level" throughout the experiment.

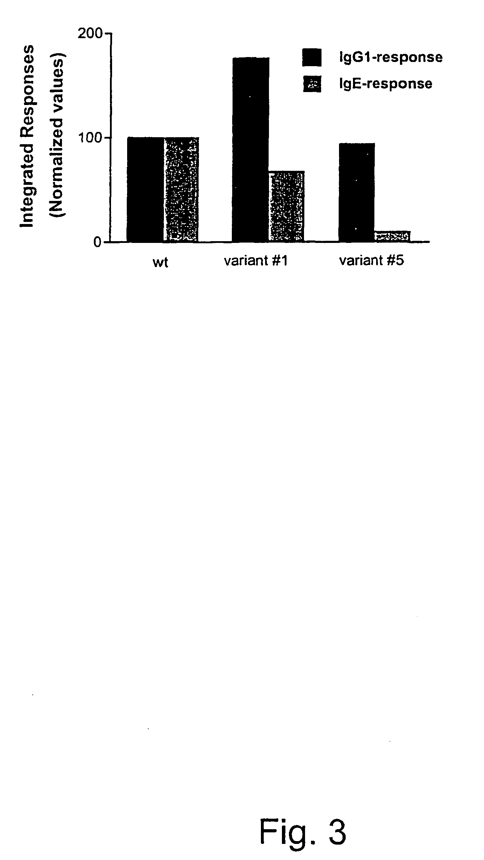

Figure 3 shows how the allergenicity of the lipase variants, determined by the integrated IgE response, decreases as the number of glycosylation sites increases. Table 2 shows how the end-point allergenicity of the lipase variants, determined by the IgE response, also decreases as the number of glycosylation sites increases. The antigenicity of the variants, determined as the IgG1 response is, however, either constant or elevated, the latter possibly being due to the antigenic properties of the glycosyl chains.

**Table 2: End-point antibody response levets in IT-rat model.**

| Enzyme | IgGl response (antigenicity) (%) | IgE response (allergenicity) (%) |
|---|---|---|
| Lipase | 100 | 100 |
| Variant #1 | 182 | 71 |
| Variant #5 | 93 | 42 |

### Example 5: SC-Mouse studies (IgGl and IgE)

The antigenic and allergenic potencies of variants #1 and #5 and of wild type lipase were compared in a mouse model with subcutaneous exposure to antigen.

The immunization protocol was similar to that of the rat studies (Example 4), except that immunizations were performed subcutaneously with a protein concentration of 0,025 mg protein/ml and that blood samples of only a 100 µl were collected from the smaller animals (female Balb/c mice, 9 weeks of age of about 20 g)

The ELISAs were performed in a manner, essentially similar to that of the rat studies (Example 4), except that anti-mouse IgG1 and anti-mouse IgE antibodies were used, and that dilution series were started from 1:160 for IgG1-ELISA and from undiluted for the IgE ELISA.

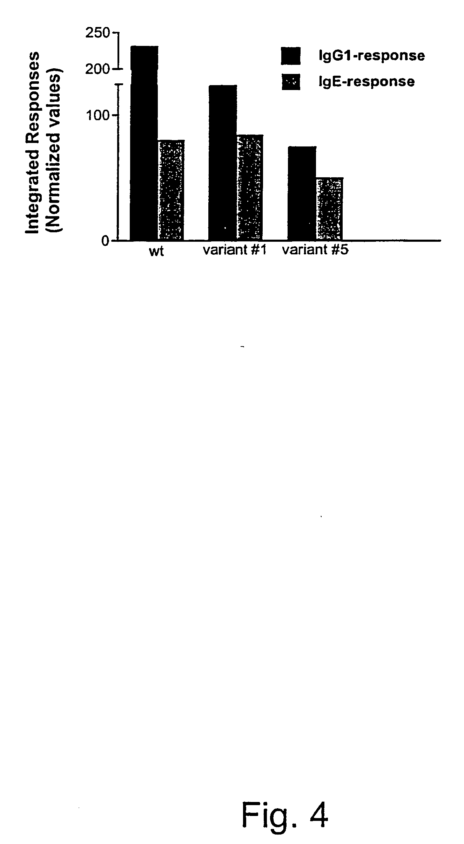

The results are shown in Figure 4. It can be seen that the allergenicity expressed as integrated antibody response levels (as is commonly accepted) is reduced for the glycosylated variants.

Thus, variant #5 show reduced allergenicity in the SC-mouse model, but it is only a limited reduction in allergenicity compared to that observed in the IT-rat model (Example 4). The difference between these two sets of results indicates that the lowered allergenicity in rats is at least partially due to increased scavenging by macrophages specific for the lung, e.g. alveolar macrophages. This is consistent with observations made in the prior art that proteins injected subcutanously have little effect on alveolar macrophages (see for example Halme et al., J. Immunotherapy with Emphasis on Tumor Immunology, vol. 15, pp 283-291, 1994 or Vasil'eva et al., Zhurnal Mikrobiologii, Epidemiologii I Imuunobiologii (10), pp. 34-36, oct. 1991) and also with observations that alveolar, but not interstitial, macrophages surpress the function of pulmonary dendritic cells as stimulators of T-cell proliferation (Armstrong LR, Christensen PJ, Paine R III et al (1994) Am J Respir Cell Mol Biol 11: 682-691 and Holt PG, Oliver J, Bilyle B et al (1993) J Exp Med: 397-407).

There is only a limited reduction in allergenicity of variant #5 when injected subcutaneously in mice. The difference between these results and those obtained by intratracheal installation of the same proteins in rats (Example 4) indicates that the lowered allergenicity in rats is at least partially due to increased scavenging by macrophages specific for the lung, e.g. alveolar macrophages. This is consistent with observations made in the prior art with respect to the extent of effect of proteins injected subcutanously on alveolar macrophages (see for example J. Immunotherapy with Emphasis on Tumor Immunology, vol. 15, pp 283-291, 1994 or Zhurnal Mikrobiologii, Epidemiologii I Imuunobiologii (10), pp. 34-36, oct. 1991).

### Example 6: Competitive ELISA.

Competitive ELISA experiments were conducted for lipase wild-type and the two variants using methods known in the art with specific polyclonal anti-lipase antiserum from rabbits and lipase wild-type as the competitor (see e.g J. Clausen, Immunochemical Techniques For The Identification And Estimation Of Macromolecules, Elsevier, Amsterdam, 1988 pp.187-188).

The wild type has, by definiton, no loss of competitivity and an endpoint inhibiton of 100%. Variant #1 had 2-fold loss of competitivity and an endpoint inhibiton of 90%, whereas variant #5 had 256-fold
loss of competitivity and an endpoint inhibition of 53%.

The results indicate that at least a part of the reduced allergenicity can be due to reduced antigenicity, consistent with the fact that glycosylation sites lip.2.-4., lip.3.0, lip.4.0 and lip.5.2 are located in the vicinity of lipase epitopes.

## Claims

1. A method of producing an N-glycosylated protein variant having a reduced allergenicity measured as a reduced IgE antibody production in animals, including man, as compared to parent protein, comprising
constructing DNA molecules encoding protein variants, said DNA molecules having at least one sub-sequence encoding an additional N-glycosylation site compared to the parent protein,
selecting a DNA molecule encoding a glycosylated protein variant, having an allergenicity reduced by at least 50% compared to the parent protein, the allergenicity being measured as a reduced IgE antibody production in rats exposed intratracheally to the variant,
introducing the DNA molecule encoding the variant into a suitable host capable of glycosylation,
culturing said host in a suitable medium, whereby said protein variant is expressed and glycosylated in the host,
recovering the glycosylated protein variant from the medium.

2. The method according to Claim 1, wherein the allergenicity, as expressed by the IgE antibody response in rats, of the glycosylated protein variant is lower than 1% of the parent protein.

3. The method according to Claims 1-2, wherein the DNA molecule has at least two subsequences encoding additional glycosylation sites.

4. The method according to any of the preceding claims, wherein the additional glycosylation site(s) is (are) introduced at a position affecting epitopes.

5. The method according to Claim 4, wherein the epitope(s) is/are identified by screening of phage display libraries.

6. The method of Claim 1, wherein the protein variants are a diversified library of variants.

7. The method according to any of the preceding claims, wherein the protein is an enzyme.

8. The method according to Claim 7, wherein the enzyme is selected from the groups consisting of proteases, lipases, phytases, polysaccharide lyases, oxidoreductases, transglutaminases, glycosyl hydrolases, and glycoseisomerases.

9. The method according to Claim 8, wherein the enzyme is a lipase.

10. The method according to Claim 9, wherein the additional glycosylation sites are chosen from N33, N37, N163 and/or N212 of the sequence for Humicola lanuginosa lipase or from the corresponding sites in homologous lipases.

## Patentansprüche

1. Verfahren zur Herstellung einer N-glycosylierten Proteinvariante mit einer im Vergleich zum Ausgangsprotein verminderten Allergenizität, gemessen als reduzierte IgE-Antikörperproduktion in Tieren, den Menschen eingeschlossen, umfassend:
- Konstruieren von DNA-Molekülen, die Proteinvarianten codieren, wobei die DNA-Moleküle, im Vergleich zu dem Ausgangsprotein, mindestens eine Subsequenz aufweisen, die eine zusätzliche N-Glycosylierungsstelle codiert,
- Selektieren eines DNA-Moleküls, das eine glycosylierte Proteinvariante codiert, mit einer im Vergleich zu dem Ausgangsprotein um mindestens 50 % verminderten Allergenizität, wobei die Allergenizität als reduzierte IgE-Antikörperproduktion in Ratten, die der Variante intratracheal ausgesetzt waren, gemessen wird,
- Einbauen des DNA-Moleküls, das die Variante codiert, in einen geeigneten Wirt, der zur Glycosylierung in der Lage ist,
- Kultivieren des Wirts in einem geeigneten Medium, wobei die Proteinvariante in dem Wirt exprimiert und glycosyliert wird,
- Gewinnen der glycosylierten Proteinvariante aus dem Medium.

2. Verfahren nach Anspruch 1, wobei die Allergenizität, ausgedrückt durch die IgE-Antikörperantwort in Ratten, der glycosylierten Proteinvariante geringer ist als 1 % des Ausgangsproteins.

3. Verfahren nach den Ansprüchen 1 bis 2, wobei das DNA-Molekül mindestens 2 Subsequenzen aufweist, die zusätzliche Glycosylierungsstellen codieren.

4. Verfahren nach einem der vorhergehenden Ansprüche, wobei die zusätzliche(n) Glycosylierungsstelle(n) in eine Epitope beeinflussende Position eingebaut wird (werden).

5. Verfahren nach Anspruch 4, wobei das (die) Epitop(e) durch Screening von Phagendisplaybibliotheken identifiziert wird (werden).

6. Verfahren nach Anspruch 1, wobei die Proteinvarianten eine diversifizierte Bibliothek von Varianten sind.

7. Verfahren nach einem der vorhergehenden Ansprüche, wobei das Protein ein Enzym ist.

8. Verfahren nach Anspruch 7, wobei das Enzym aus der Gruppe ausgewählt ist, bestehend aus Proteasen, Lipasen, Phytasen, Polysaccharidlyasen, Oxidoreductasen, Transglutaminasen, Glycosylhydrolasen, und Glycoseisomerasen.

9. Verfahren nach Anspruch 8, wobei das Enzym eine Lipase ist.

10. Verfahren nach Anspruch 9, wobei die zusätzlichen Glycosylierungsstellen aus N33, N37, N163 und/oder N212 der Sequenz für Humicola lanuginosa-Lipase oder aus den entsprechenden Stellen in homologen Lipasen ausgewählt sind.

## Revendications

1. Méthode pour produire un variant de protéine N-glycosylée ayant une allergénicité réduite mesurée en tant que production d'anticorps IgE réduite chez l'animal, y compris l'homme, par rapport à la protéine parente, comprenant
la construction de molécules d'ADN codant des variants de protéine, lesdites molécules d'ADN ayant au moins une sous-séquence codant un site de N-glycosylation supplémentaire par rapport à la protéine parente,
la sélection d'une molécule d'ADN codant un variant de protéine glycosylée, ayant une allergénicité réduite d'au moins 50 % par rapport à la protéine parente, l'allergénicité étant mesurée en tant que production d'anticorps IgE réduite chez des rats exposés de manière intratrachéale au variant,
l'introduction de la molécule d'ADN codant le variant dans un hôte adapté capable de glycosylation,
la culture dudit hôte dans un milieu adapté, par quoi ledit variant de protéine est exprimé et glycosylé dans l'hôte,
la récupération du variant de protéine glycosylée à partir du milieu.

2. Méthode selon la revendication 1, dans laquelle l'allergénicité, telle qu'exprimée par la réponse en anticorps IgE chez des rats, du variant de protéine glycosylée est inférieure à 1 % de la protéine parente.

3. Méthode selon les revendications 1-2, dans laquelle la molécule d'ADN a au moins deux sous-séquences codant des sites de glycosylation supplémentaires.

4. Méthode selon l'une quelconque des revendications précédentes, dans laquelle le(s) site(s) de glycosylation supplémentaire(s) est(sont) introduit(s) au niveau d'une position affectant des épitopes.

5. Méthode selon la revendication 4, dans laquelle le(s) épitope(s) est(sont) identifié(s) par le criblage de banques de présentation par les phages.

6. Méthode selon la revendication 1, dans laquelle les variants de protéine correspondent à une banque diversifiée de variants.

7. Méthode selon l'une quelconque des revendications précédentes, dans laquelle la protéine est une enzyme.

8. Méthode selon la revendication 7, dans laquelle l'enzyme est choisie dans les groupes constitués de protéases, lipases, phytases, polysaccharide lyases, oxydoréductases, transglutaminases, glycosyl hydrolases et glycoseisomérases.

9. Méthode selon la revendication 8, dans laquelle l'enzyme est une lipase.

10. Méthode selon la revendication 9, dans laquelle les sites de glycosylation supplémentaires sont choisis parmi N33, N37, N163 et/ou N212 de la séquence pour la lipase de *Humicola lanuginosa* ou parmi les sites correspondants dans des lipases homologues.
